# EUROPÄISCHE PATENTSCHRIFT

(11) **EP 1 289 574 B1**
(45) Veröffentlichungstag und Bekanntmachung des Hinweises auf die Patenterteilung: **13.06.2007**
(21) Anmeldenummer: 01953164.9
(22) Anmeldetag: 29.05.2001
(51) Int. Cl.: A61L 27/24

(54) **VERFAHREN ZUR KULTIVIERUNG VON KNORPELERSATZ UND BIOMATRIX NACH DIESEM VERFAHREN HERGESTELLT**
CARTILAGE REPLACEMENT AND METHODS FOR THE PRODUCTION THEREOF
CARTILAGE ARTIFICIEL ET PROCEDES PERMETTANT DE LE PRODUIRE

(30) Priorität: 31.05.2000 DE 10026789
(43) Veröffentlichungstag der Anmeldung: 12.03.2003
(60) Teilanmeldung: 07002971.5
(73) Patentinhaber: Arthro Kinetics AG, 73728 Esslingen (DE)
(72) Erfinder: NOLL, Michaela, 70597 Stuttgart (DE); SCHANDAR, Markus, 71720 Oberstenfeld (DE); GRAEVE, Thomas, 70372 Stuttgart (DE)
(74) Vertreter: Schrell, Andreas
(86) Internationale Anmeldenummer: PCT/EP2001/006073
(87) Internationale Veröffentlichungsnummer: WO 2001/092473

(56) Entgegenhaltungen:
- EP-A- 0 896 825
- EP-A- 1 027 897
- WO-A-00/78370
- WO-A-95/33821
- WO-A-98/51317
- US-A- 5 786 217
- US-A- 5 834 029
- BUIJA J ET AL: "SYNTHESIS OF HUMAN CARTILAGE USING ORGANOTYPIC CELL CULTURE" ORL, BASEL, CH, Bd. 55, 1993, Seiten 347-351, XP001016352 ISSN: 0301-1569
- SITTINGER M ET AL: "ENGINEERING OF CARTILAGE TISSUUE USING BIORESORBABLE POLYMER CARRIERS IN PERFUSION CULTURE" BIOMATERIALS, ELSEVIER SCIENCE PUBLISHERS BV., BARKING, GB, Bd. 15, Nr. 6, 1. Mai 1994 (1994-05-01), Seiten 451-456, XP002024594 ISSN: 0142-9612
- FUJISATO TOSHIA ET AL: "Effect of basic fibroblast growth factor on cartilage regeneration in chondrocyte-seeded collagen sponge scaffold." BIOMATERIALS, Bd. 17, Nr. 2, 1996, Seiten 155-162, XP002184080 ISSN: 0142-9612
- SCHUMAN LEIN ET AL: "Chondrocyte behaviour within different types of collagen gel in vitro." BIOMATERIALS, Bd. 16, Nr. 10, 1995, Seiten 809-814, XP004032926 ISSN: 0142-9612

## Beschreibung

Die Erfindung betrifft Verfahren zur Kultivierung von differenzierten und dedifferenzierten Knorpelzellen, einen Knorpelersatz, eine Biomatrix, ein Zell-Matrix-Knorpel-System sowie Verfahren zu deren Herstellung.

Gelenkerkrankungen stellen eine weitverbreitete und mit einer Vielzahl von Beschwernissen für die betroffenen Personen verbundene Krankheit dar. So spielen Knochen- und Knorpeldefekte eine zentrale Rolle bei der Pathogenese von Arthrose, aber auch bei posttraumatischen Zuständen und ausgelockerten Endoprothesen. Derartige Defekte können durch Einsatz autologer oder homologer Knochen beziehungsweise Knorpel beziehungsweise entsprechender Ersatzmaterialien therapiert werden. Während autologe Materialien nicht unbegrenzt zur Verfügung stehen, müssen bei homologen Transplantaten infektiologische Gesichtspunkte berücksichtigt werden.

Das Hauptproblem degenerativer oder traumatischer Gelenkerkrankungen besteht darin, dass der geschädigte Gelenkknorpel nur eine geringe Fähigkeit zu Regeneration zeigt. Es ist bekannt, derartige Knorpeldefekte mittels der autologen Chondrocytentransplantation (ACT) als biologischem Therapieverfahren zu behandeln. Mit diesem Therapieverfahren konnte erstmals die Neubildung von hyalinem Knorpel in einem Gelenkknorpeldefekt nachgewiesen werden.

Das Prinzip dieser Methode basiert darauf, in vitro kultivierte Knorpelzellen des Patienten nach Debridement des degenerierten Knorpels unter einen auf den Defekt genähten Periostlappen zu spritzen (Peterson-Methode). Das Verfahren ist technisch anspruchsvoll und wirft methodische Probleme auf; so ist beispielsweise der längerfristige Verbleib der Knorpelzellen im Defekt nicht garantiert. Des Weiteren ist unklar, ob die kultivierten Knorpelzellen zum Zeitpunkt der Transplantation in der Lage sind, die für die dauerhafte Defektfüllung benötigte Matrix zu bilden. Die transplantierten Zellen befinden sich zumeist in einem dedifferenzierten Zustand und weisen morphologische und physiologische Gemeinsamkeiten mit Fibroblasten auf.

Aus der DE 197 21 661 A1 ist es bekannt, neuartige Materialien für den Ersatz von Knochen oder Knorpeln, die sich durch eine spezifische Struktur an sich bekannter Werkstoffe auszeichnen, zu verwenden. Es handelt sich um ein Knochen- beziehungsweise Knorpelimplantat auf Basis eines dreidimensionalen Gitters, das im Wesentlichen aus einer Vielzahl von regelmäßig angeordneten Stäben aus einem teilweise oder vollständig bioresorbierbaren Werkstoff besteht. Die Stäbe bilden eine geometrische dreidimensionale Struktur aus, wobei diese Struktur in Bezug auf Elastizität und Festigkeit auf das Gewebe abgestimmt ist, das es im Körper des Patienten ersetzen soll. Die Stäbe können aus Poly-D-Lactiden, Poly-L-Lactiden, Poly-DL-Lactiden, Hydroxylapatiten, Calciumphosphaten oder Mischungen dieser Stoffe bestehen, die im Wesentlichen Calciumphosphate beziehungsweise Hydroxylapatite, Kollagen, Agar oder Gelatine enthalten.

Die WO 99/08728 offenbart ein osteoinduktives oder chondroinduktives Faktorengemisch, das eingebettet in Nanosphären vorliegt. Offenbart wird auch ein System umfassend eine bioabbaubare Matrix, enthaltend z.B. Kollagen Typ I oder Typ II und darin eingebettet die von den Nanosphären umhüllten Faktoren. Die Nanosphären sind als Polymerpartikel ausgeführt.

Aus der WO 95/33821 ist es bekannt, dreidimensionale Strukturen aus Kollagen herzustellen, wobei die interstitiellen Bereiche durch z.B. Fibroblasten oder Chondrocyten überbrückt werden und eine Kultivierung dieses Netzwerkes in einem Kulturmedium durchgeführt wird. In dieser Druckschrift wird die Kultivierung der genannten Zellen auf diesem dreidimensionalen Netzwerk beschrieben ebenso wie die Verwendung dieses künstlichen Gewebes als Knorpelersatz.

Die WO 98/17791 beschreibt die Isolierung und die Verwendung von Vorläufer-Chondrocyten, die in Kultur methodisch vermehrt und zur Verwendung therapeutisch einsetzbaren Knorpelgewebes verwendet werden können. Auch hier wird die Kultivierung von Chondrocyten auf einem dreidimensionalen Netzwerk beschrieben, wobei die interstitiellen Räume durch die Chondrocyten überbrückt werden.

Die WO 99/00152 offenbart ein Verfahren zur Herstellung eines bioartifiziellen Transplantates, wobei aus einem allogenen oder xenogenen Gewebe alle antigen-reaktiven Zellen durch enzymatische oder chemische Behandlung entfernt werden und das so gewonnene zellfreie, nicht-denaturierte Material mit gewünschten autologen Zellen besiedelt wird, wodurch ein unmittelbar einsatzbereites Transplantat erhalten wird.

Bei den bekannten Verfahren wirkt sich nachteilig aus, dass transplantierte oder kultivierte Knorpelzellen ihre ursprüngliche Syntheseleistung nicht wieder erreichen.

Die in der Interzellularsubstanz des Knorpels eingelagerten Knorpelzellen -die Chondrocyten- dedifferenzieren nämlich unter in vitro Kulturbedingungen im Laufe der Kultivierungsdauer. Während primäre Knorpelzellen (P0 Kultur) nach ihrer Isolation aus Knorpelgewebe noch knorpelzelltypische Syntheseleitungen, wie die Bildung von Kollagen Typ II, Typ IX und Typ XI, zeigen, verlieren kultivierte Zellen diese typischen Eigenschaften während weiterer Passagen (P1-PX) in vitro.

Knorpelzellen sind im Gegensatz zu anderen Zellen überdies schwer zu kultivieren. Kultivierte Knorpelzellen befinden sich in einem dedifferenzierten Zustand und ähneln morphologisch und physiologisch den Fibroblasten. Vor allem bei größeren Defekten ist jedoch die Vermehrung der autologen Chondrocyten durch Kultivierung und Passagierung für die Herstellung ausreichender Mengen an Transplantationsmaterial aus kleinen Proben notwendig.

Die Dedifferenzierung der Knorpelzellen kann bisher nur durch die Zugabe von zusätzlichen Wachstumsstimulanzien verhindert werden, was die Verwendung dieser Kulturen im in vivo Bereich erschwert, da die Wachstumsstimulanzien mit dem Immunsystem des Empfängerorganismus nachteilig wechselwirken können. Durch die bekannten Verfahren ist es des Weiteren nicht gewährleistet, dass die Zellen auch nach mehreren Passagen während der Kultivierung ihren natürlichen oder weitestgehend natürlichen Stoffwechsel aufrecht erhalten.

Die bekannten dreidimensionalen Strukturen für die Kultivierung von Knorpelzellen können in den bisherigen Verfahren zudem nicht spezifisch dem jeweiligen Knorpeldefekt angepasst werden. Es ist weiterhin nicht gewährleistet, dass die transplantierten Knorpelzellen an der Stelle des Defektes dauerhaft und differenziert verbleiben und damit die Regeneration des Knorpels ermöglichen.

Tatsächlich gibt es bisher kein Transplantationsverfahren, welches einen dauerhaften Verbleib von transplantierten Knorpelzellen an der Stelle des Defektes und eine damit verbundene Regeneration des Knorpels gewährleistet.

Das der vorliegenden Erfindung zugrunde liegende technische Problem besteht also darin, Knorpeltransplantate sowie Verfahren und Mittel zu deren Herstellung bereit zu stellen, die eine verbesserte Behandlung von Knorpelerkrankungen, insbesondere eine verbesserte Heilung beziehungsweise Regeneration des behandelten Knorpeldefektes erlauben.

Die Erfindung löst das ihr zugrunde liegende Problem insbesondere durch die Bereitstellung eines Verfahrens zur Redifferenzierung und/oder Vermehrung von dedifferenzierten Knorpelzellen, wobei die dedifferenzierten Knorpelzellen in einer dreidimensionalen gelartigen Biomatrix kultiviert werden, dort redifferenzieren und ihre zelltypischen Stoffwechselleistungen wieder aufnehmen. Die erfindungsgemäße Biomatrix enthält ein aus einer, vorzugsweise frischen, Kollagenlösung neu konstituiertes Kollagengerüst einer Konzentration von mindestens 1,5 mg Kollagen/ml Biomatrix, vorzugsweise 1,5 bis 4 mg Kollagen/ml Biomatrix. Dieses Kollagengerüst wird aus einer, vorzugsweise zellfreien, sauren Kollagenlösung, vorzugsweise mit einem pH-Wert von 0,1 bis 6,9, vorzugsweise 2,0 bis 5,0, bevorzugt 3,0 bis 4,5, insbesondere 3,2 bis 4,2 und besonders bevorzugt 3,8, gewonnen, die bei 2 bis 10°C, vorzugsweise 4°C, präpariert und gelagert wurde. Diese Kollagenlösung wird zur Herstellung einer zellfreien Biomatrix bei 2 bis 10°C, vorzugsweise bei 4°C, mit einer Lösung aus Medium, insbesondere einem konventionellen Zellkulturmedium, Puffer, zum Beispiel Hepes-Puffer, und Serum, insbesondere humanem autologem Serum, versetzt und durch Erhöhung der Temperatur auf zum Beispiel Raumtemperatur oder 37°C geliert. Zur Herstellung einer zellhaltigen Biomatrix werden, vorzugsweise vorkultivierte, Zellen, zum Beispiel Knorpelzellen oder Vorläuferknorpelzellen, in die Lösung aus Medium, Puffer und Serum eingebracht und dann bei 2 bis 10°C, vorzugsweise 4°C, mit der ebenfalls 2 bis 10°C, vorzugsweise 4°C, temperierten Kollagenlösung versetzt. Die so in die Biomatrix eingebetteten Zellen können dann kultiviert und gegebenenfalls wieder herausgelöst werden. Anschließend wird bei zum Beispiel Raumtemperatur oder 37°C geliert und die Biomatrix mit Zellkulturmedium überschichtet. Das erfindungsgemäße Verfahren gestattet es vorteilhafterweise, eine Zwischenkultivierung der Zellen in einer erfindungsgemäßen Biomatrix durchzuführen, wobei die Chondrocyten, zum Beispiel der P2-Kultur, in der dreidimensionalen Biomatrix zu der erneuten Synthese von zelltypischen Matrixproteinen, insbesondere Kollagen II stimuliert werden, wohingegen in bekannten Kulturen keine Kollagenbildung nachgewiesen werden kann. Vorteilhafterweise können die Zellen anschließend wieder aus der Biomatrix herausgelöst und kultiviert werden. Durch die erfindungsgemäß ermöglichte Redifferenzierung von dedifferenzierten Knorpelzellen ist es möglich, Chondrocyten über einen längeren Zeitraum hin zu kultivieren und/oder zu vermehren, ohne dass die Zellen die spezifischen, für den Knorpelaufbau notwendigen, Syntheseleistungen verlieren. Mit Vorteil kann so auch bei einer geringen Ausgangsmenge an Knorpelgewebe genügend Zellmaterial für die Herstellung von Knorpeltransplantaten zur Verfügung gestellt werden.

Im Zusammenhang mit der vorliegenden Erfindung wird unter dem Begriff Kultivieren von Zellen ein, vorzugsweise in vitro stattfindendes, Aufrechterhalten der Lebensfunktionen von Zellen in einer geeigneten Umgebung, zum Beispiel unter Zu- und Abfuhr von Stoffwechseledukten und -produkten, verstanden, insbesondere auch eine Vermehrung der Zellen.

Im Zusammenhang mit der vorliegenden Erfindung werden unter Knorpelzellen oder Chrondrocyten natürlicherweise vorkommende oder gentechnisch veränderte Knorpelzellen oder ihre Vorläufer verstanden, die tierischer oder menschlicher Herkunft sein können.

In einer weiteren Ausführungsform der Erfindung ist vorgesehen, differenzierte Knorpelzellen, insbesondere unter Erhalt ihrer Differenzierung, in einer wie vorstehend charakterisierten dreidimensionalen Biomatrix zu kultivieren. Vorteilhafterweise werden die Stoffwechselleistungen der Primärkultur erhalten, ohne dass eine Dedifferenzierung der differenzierten Knorpelzellen erfolgt. Die Erfindung stellt also auch ein Kultivierungsverfahren für Chondrocyten bereit, das eine Unterdrückung der Redifferenzierung umfasst.

In einer weiteren vorteilhaften Ausgestaltung der Erfindung ist vorgesehen, dass in ihrer Funktion, ihrer Morphologie und/oder ihrem Differenzierungsstatus zu überprüfende Knorpelzellen in eine genannte dreidimensionale Biomatrix eingebracht, kultiviert und dabei und/oder danach überprüft werden. Vorteilhafterweise kann so beispielsweise der Stoffwechsel der Knorpelzellen in vitro untersucht werden, bevor die Knorpelzellen in vivo eingesetzt werden. Die Überprüfung kann zum Beispiel die Messung von Stoffwechselleistungen ebenso wie eine morphologische oder Funktionsüberprüfung sein.

Die Erfindung betrifft daher auch Screening- und Diagnoseverfahren, wobei Knorpelzellen gemäß den vorstehend beschriebenen Verfahren kultiviert und dabei oder anschließend untersucht werden können, zum Beispiel auf physiologische, morphologische und/oder molekularbiologische Parameter. Insbesondere können dabei degenerative oder traumatische Gelenkerkrankungen beziehungsweise deren Abwesenheit nachgewiesen werden. Im Rahmen dieser Verfahren können auch die Auswirkungen von potentiellen Medikamenten und/oder Krankheitserregern, Antigenen oder ähnlichem auf die kultivierten Zellen untersucht werden, zum Beispiel in drug screening Verfahren. Dabei werden in bevorzugter Ausführung die Zellen in An- und Abwesenheit des zu untersuchenden Agens kultiviert und die beobachteten Auswirkungen miteinander verglichen.

In einer weiteren vorteilhaften Ausführungsform der Erfindung werden die Knorpelzellen im Anschluss an die Kultivierung in der dreidimensionalen Biomatrix aus dieser herausgelöst, zum Beispiel mittels Kollagenasebehandlung und anschließendem Aufkonzentrieren, und in einer herkömmlichen zweidimensionalen oder dreidimensionalen Zellkultur weiter kultiviert. Vorteilhafterweise können so die Vorteile der zweidimensionalen und dreidimensionalen Kultivierung kombiniert werden.

In einer besonders vorteilhaften Ausführungsform der Erfindung werden die Knorpelzellen in eine erfindungsgemäße dreidimensionale Biomatrix eingebracht und in dieser so kultiviert, dass anschließend ein transplantierbarer Knorpelersatz, auch als Knorpeltransplantat bezeichnet, gewonnen werden kann. Dieser Knorpelersatz kann in bevorzugter Ausführung ein Gelenkknorpelersatz sein. Mit Vorteil kann so beispielsweise während der Phase des Kultivierens der Zellen bereits die Biomatrix der Form des Knorpeldefektes angepasst werden.

In einer vorteilhaften Ausführungsform betrifft die Erfindung auch ein vorgenanntes Verfahren zur Herstellung eines Knorpelersatzes, insbesondere Gelenkknorpelersatzes, wobei bevorzugt die Knorpelzellen nach Kultivierung in der dreidimensionalen Biomatrix aus dieser herausgelöst, vorzugsweise mittels Kollagenasebehandlung und Abzentrifugieren, und in -vorzugsweise- höherer Zelldichte in einer herkömmlichen zwei- oder dreidimensionalen Zellkultur weiterkultiviert werden, wobei ein transplantierbarer Knorpelersatz, insbesondere Gelenkknorpelersatz, gewonnen werden kann.

Die Erfindung betrifft auch einen Knorpelersatz, insbesondere Gelenkknorpelersatz, der nach dem erfindungsgemäßen Verfahren und einem gegebenenfalls anschließenden oder/und vorausgehenden Kultivierungsverfahren herkömmlicher Art hergestellt wurde.

Die Erfindung betrifft auch eine, vorzugsweise gelartige, Biomatrix, in der die vorgenannten Kultivierungsverfahren durchgeführt werden können, und zwar eine Biomatrix sowohl ohne Knorpelzellen als auch eine Biomatrix mit Knorpelzellen. Im letzten Fall wird die Kombination aus Biomatrix und darin kultivierten differenzierten oder redifferenzierten Knorpelzellen auch als Zell-Matrix-Knorpel-System oder Biotransplantat bezeichnet. Dieses Biotransplantat kann direkt zur Therapie von Knorpelerkrankungen oder -defekten eingesetzt werden.

Erfindungsgemäß wird unter einer Biomatrix eine Gelstruktur enthaltend Kollagen, Zellkulturmedium, Serum und Puffer, insbesondere Hepes-Puffer, verstanden. Die für die Herstellung der Biomatrix verwendete Kollagenlösung stellt eine Lösung dar, die einen hohen Anteil an nicht denaturiertem, nativen Kollagen in saurem, wässrigen Medium enthält, insbesondere mit einem pH-Wert von 0,1 bis 6,9, vorzugsweise 2,0 bis 5,0, insbesondere 3,0 bis 4,5, bevorzugt 3,2 bis 4,2 und besonders bevorzugt 3,8, zum Beispiel in Essigsäure, insbesondere 0,1 %iger Essigsäurelösung. Unter einem hohen Anteil wird ein Anteil von nicht denaturiertem Kollagen am Gesamtkollagen in Lösung von ≥ 50 %, insbesondere ≥ 60, ≥ 70, ≥ 80, ≥ 90 oder ≥ 95, insbesondere ≥ 99 % verstanden. In bevorzugter Ausführung wird kein lyophilisiertes Kollagen verwendet. Der Kollagengehalt der Lösung liegt vorteilhafterweise zwischen 3 mg Kollagen/ml Lösung bis 8 mg Kollagen/ml Lösung, vorzugsweise 6 mg Kollagen/ml Lösung. Vorteilhafterweise wird in bevorzugter Ausführungsform Kollagen verwandt, das nach Isolierung aus zum Beispiel Rattenschwänzen in 0,1 %iger Essigsäure für 3 bis 14 Tage bei 4°C unter Rühren inkubiert wurde und wobei nicht gelöste Kollagenanteile abzentrifugiert wurden. Als Zellkulturmedium wird vorteilhafterweise DMEM (Dulbecco's Modified Eagle Medium) verwendet. Es kann aber auch jedes beliebige andere Zellkulturmedium verwendet werden, welches die Kultivierung von Knorpelzellen erlaubt. Als Serum wird in bevorzugter Ausführung vorteilhafterweise autologes humanes Serum verwendet und als Puffer zum Beispiel Hepes. In bevorzugter Ausführung wird eine 3 M Konzentration von Hepes in dieser Lösung eingestellt. Der pH-Wert der Lösung aus Zellkulturmedium, Puffer und Serum beträgt in bevorzugter Ausführung 7,5 bis 8,5, zum Beispiel 7,6 bis 8,2, insbesondere 7,8. Selbstverständlich kann die Biomatrix auch weitere Faktoren, wie Wachstumsfaktoren, Adhäsionsmittel, Antibiotica, Selektionsmittel oder ähnliche, enthalten.

Die Erfindung betrifft daher auch Verfahren zur Herstellung einer Biomatrix, wobei in einem ersten Schritt frisches Kollagen, beispielsweise aus Rattenschwänzen, hergestellt wird, indem aus kollagenhaltigem Gewebe isolierte Kollagenfasern in Pufferlösung gesammelt, in Alkohol oberflächlich desinfiziert und anschließend in Pufferlösung gewaschen und anschließend in eine saure Lösung eines pH-Wertes von 0,1 bis 6,9, vorzugsweise 2,0 bis 5,0, besonders bevorzugt 3,0 bis 4,0, insbesondere 3,3, zum Beispiel eine 0,1 %ige Essigsäurelösung, überführt werden. Anschließend wird in einem weiteren Schritt das in der Lösung befindliche Kollagen bei 2 bis 10°C, insbesondere 4°C, für einige Tage, zum Beispiel 3 bis 14 Tage, gerührt, die nicht gelösten Kollagenanteile werden abzentrifugiert und eine fertige Kollagenlösung mit einem Kollagengehalt von 3 mg/ml bis 8 mg/ml bei 2 bis 10°C, zum Beispiel 4°C, aufbewahrt. Selbstverständlich ist es möglich, die Lösung in gefrorenem Zustand zu lagern, zum Beispiel bei -10°C bis -80°C, insbesondere -20°C. In einem dritten Schritt wird diese Kollagenlösung mit einer Lösung eines pH-Wertes von 7,5 bis 8,5, bevorzugt 7,6 bis 8,2, insbesondere 7,8, enthaltend doppeltkonzentriertes Zellkulturmedium, Serum und Puffer, im Verhältnis von vorzugsweise 1:1 gemischt und so eine Biomatrix erhalten, die einen pH-Wert von 7,0 bis 7,8, bevorzugt 7,4, aufweist. Zur Herstellung eines Biotransplantats wird die Lösung aus doppeltkonzentriertem Zellkulturmedium, Serum und Puffer mit vorkultivierten und abzentrifugierten Knorpelzellen gemischt, wobei vorzugsweise 2x10⁴ bis 2x10⁷ Zellen pro ml, bevorzugt 2x10⁶ Zellen, verwendet werden. Diese Lösung eines pH-Wertes von 7,5 bis 8,5, bevorzugt 7,6 bis 8,2, insbesondere 7,8 wird anschließend im Verhältnis 1:1 mit der vorgenannten Kollagenlösung bei 2 bis 10°C, insbesondere 4°C, gemischt. Anschließend wird die Gellösung in Kulturgefäße pipettiert und nach Gelieren bei 37°C mit Medium überschichtet. Sodann wird das Biotransplantat 3 bis 8 Tage kultiviert, um anschließend für Transplantationen zur Verfügung zu stehen.

Das Biotransplantat kommt in Kulturgefäßen in den Operationssaal und kann dann durch den Mediziner durch mechanische Bearbeitung, zum Beispiel mit einem Messer, in Größe, Dicke und Form dem Defekt angepasst werden. Anschließend wird das Biotransplantat, zum Beispiel mittels Gewebekleber, insbesondere Fibrinkleber, in dem Defekt fixiert. Nach circa 5 Minuten ist das Biotransplantat im Defekt fest verankert. Die Operation kann auch arthroskopisch durchgeführt werden, da das Biotransplantat flexibel ist.

Ein weiterer Einsatz des Biotransplantats stellt eine Kombination des erfindungsgemäßen Biomatrixplantats mit Knochen dar. Dabei wird das Biotransplantat zum Beispiel auf autologe Knochenzylinder aus dem Beckenkamm, zum Beispiel mittels Fibrinkleber, aufgebracht. Erfindungsgemäß wird insbesondere die Therapie von Osteochondrosis dissecans und Defekte am Hüftkopf bei cerebralparetisch bedingter Hüftluxation sowie die Sanierung von traumatischen und degenerativen Läsionen im Kniegelenk ermöglicht. Durch frühzeitige und kausale Therapie kann erfindungsgemäß mit der neuen Methode ein Fortschreiten der traumatisch oder degenerativ bedingten Gelenkerkrankungen aufgehalten werden. Für die Behandlung der Arthrose beim jüngeren Menschen steht erfindungsgemäß ein einzigartiges und neues Therapieverfahren zur Verfügung, das den endoprothetischen Gelenkersatz erheblich hinauszögert, im Einzelfall auch überflüssig macht. Auch die Therapie entzündlich bedingter Gelenkdestruktion ist als Anwendung des erfindungsgemäßen Verfahrens ermöglicht.

Die Entwicklung der erfindungsgemäßen gelartigen Biomatrix, in die autologe Zellen exakt definiert eingebettet werden können, ermöglicht erstmals die Transplantation einer definierten Menge von Knorpelzellen in einer mechanisch formbaren und belastbaren Matrix. Der erfindungsgemäße Zell-Matrix-Knorpel-System, aufgebaut aus erfindungsgemäßer Biomatrix und Knorpelzellen, kann in beliebiger Größe und Dicke hergestellt werden und durch mechanische Bearbeitung dem Defekt genau angepasst werden. Das erfindungsgemäße Biotransplantat bietet außerdem den Vorteil, dass im Gegensatz zur ACT eine zusätzliche Periostentnahme an der Tibia des Patienten entfällt. Durch die Kultivierung des erfindungsgemäßen Zell-Matrix-Knorpel-Systems in vitro ist es möglich, die Knorpelzellen vor der Transplantation hinsichtlich ihrer Matrixproduktion zu untersuchen. Dies ist eine wichtige Voraussetzung für die Herstellung von Knorpeltransplantaten, ihre Prüfung auf Funktionalität und für die Qualitätssicherung. So konnte gezeigt werden, dass die neue Synthese typischer Knorpelproteine wie zum Beispiel Kollagen Typ II, die zur Primärstabilität des Transplantats dienen, von den autologen Knorpelzellen in der Matrix in vitro gebildet werden. Erfindungsgemäß konnte durch Versuche am Kniegelenk von Minipigs gezeigt werden, dass im Vergleich zum Leerdefekt der Transplantation von Leermatrix ohne Knorpelzellen oder von Knorpelzellsuspensionen nach Peterson die erfindungsgemäßen Zell-Matrix-Knorpel-Systeme im Gelenkknorpel äußerst gut einheilen, den Defekt vollständig ausfüllen und zudem druckstabil sind.

Schließlich betrifft die Erfindung auch eine Biomatrix zur Verwendung in einem der vorgenannten Verfahren.

Die Erfindung betrifft auch Verfahren zur Behandlung von Knorpelerkrankungen oder -defekten, insbesondere degenerativer, entzündlicher und/oder traumatischer Gelenkerkrankungen, wobei ein gemäß der vorliegenden Erfindung hergestellter Knorpelersatz, Biotransplantat, und/oder gemäß der Erfindung kultivierte Knorpelzellen, in erkrankte beziehungsweise geschädigte Gewebe oder Knorpel- beziehungsweise Knochenbereiche eingesetzt, beziehungsweise gegen die erkrankten oder defekten Bereiche ausgetauscht wird beziehungsweise werden.

Die Erfindung wird anhand von Beispielen näher erläutert.

### Beispiel 1:

### Kollagenherstellung

Zur Herstellung einer Kollagenlösung wird kollagenhaltiges Gewebe, wie zum Beispiel Sehnen aus Rattenschwänzen, verwendet. Alle Arbeiten werden unter sterilen Bedingungen mit sterilen Materialien durchgeführt. Die Rattenschwänze werden hierfür nach Lagerung bei -20°C 5 Minuten in 70 %igem Alkohol oberflächlich desinfiziert. Anschließend wird die Haut der Rattenschwänze abgezogen und die einzelnen Kollagenfasern herausgelöst. Bei Verwendung anderer Ausgangsgewebe können gegebenenfalls vorhandene Zellen schonend durch mechanische, enzymatische oder chemische Behandlung entfernt werden. Die Kollagenfasern werden in phosphatgepufferter Salinelösung (PBS) (pH 7,2) gesammelt, in 70 %igem Alkohol für 10 Minuten oberflächlich desinfiziert und danach mit PBS gewaschen. Das Gewicht der Kollagenfasern wird bestimmt und die Fasern werden in eine 0,1 %ige Essigsäurelösung überführt (circa 8 bis 12 mg/ml). Für 3 bis 14 Tage wird dieser Ansatz bei 4°C gerührt und anschließend die nicht gelösten Kollagenteile zentrifugiert (1000 rpm, 1 Stunde, 8°C). Die fertige Kollagenlösung hat in bevorzugter Ausführung einen Kollagengehalt von 3 mg/ml bis 8 mg/ml. Das Kollagen liegt also als Ausgangsmaterial, in hohem Anteil, wie vorstehend definiert, in Lösung und nicht in Faser-, Gerüst- oder Matrixform vor. Vorzugsweise ist die erhaltene Kollagenlösung zellfrei und aus frischem, nicht lyophilisiertem Kollagen hergestellt.

### Beispiel 2:

### Herstellung Biomatrix beziehungsweise Biotransplantat

Alle Arbeiten werden unter sterilen Bedingungen mit sterilen Materialien durchgeführt.

| | |
|---|---|
| Kollagenlösung: | min. 3 mg/ml in Essigsäure |
| Pufferlösung: | 77,5 ml doppeltkonzentriertes Medium (zum Beispiel DMEM) |
| | 20 ml Serum |
| | 2,5 ml Hepes-Lösung (3 M, pH 7,8) |

Beide Lösungen werden bei 4°C aufbewahrt.

Die Biomatrix besteht aus einer Kollagenlösung in 0,1 %iger Essigsäure (Kollagen aus Rattenschwanz mit einem Kollagengehalt von 3 mg/ml bis 8 mg/ml, bevorzugt 6 mg/ml) und einer Pufferlösung aus doppeltkonzentriertem Medium, Serum und Hepes-Lösung. Kurz nach Mischen dieser beiden Komponenten im Verhältnis von vorzugsweise 1:1 geliert, bei Temperaturen oberhalb von 4°C, zum Beispiel Raumtemperatur, eine dreidimensionale neu konstituierte Kollagenstruktur aus.

Zur Herstellung des Biotransplantats, also einer zellhaltigen Biomatrix, werden in üblicher Weise vorkultivierte und abzentrifugierte (1000 rpm, 10 min., RT) 2x10⁴ bis 2x10⁷ Knorpelzellen/ml in der Pufferlösung, bevorzugt 2x10⁶, aufgenommen, suspendiert und mit gleichen Teilen der Kollagenlösung bei 4°C gemischt. Diese Gellösung wird in Kulturgefäße pipettiert und nach Gelieren bei 37°C zu einer neu konstituierten Kollagenstruktur mit darin eingebetteten Knorpelzellen mit Medium überschichtet. Das Material steht dann nach 3 bis 8 Tagen Kulturdauer für die Transplantation zur Verfügung.

Sofern gewünscht, können die Zellen durch Kollagenasebehandlung und anschließendes Abzentrifugieren aus der Biomatrix wieder freigesetzt und gewonnen werden. Die erhaltenen differenzierten Zellen können Ausgangspunkt weiterer Kultivierungen sein.

### Beispiel 3:

### Transplantation am offenen Gelenk und arthroskopische Applikation

Sowohl bei der Transplantation am offenen Gelenk als auch bei der arthroskopischen Applikation wird der Zell-Matrix Knorpel zunächst in seiner Größe und Form dem Knorpeldefekt mechanisch angepasst. Anschließend wird das Transplantat in den Defekt eingebracht und dort mittels Fibrinklebers fixiert. Da es sich bei dem beschriebenen Zell-Matrix Knorpel-System um einerseits flexibles, aber dennoch formstabiles Material handelt, ist es möglich, dieses Biotransplantat in den röhrenförmigen Arthroskopieinstrumenten quasi einzurollen, und sie so in den Defekt einzubringen. Durch ihre Formstabilität nehmen die Transplantate am Bestimmungsort wieder ihre ursprüngliche Form an und können nun mit Fibrinkleber im Defekt befestigt werden.

### Beispiel 4:

### Ergebnisse von PCR-Untersuchungen

In der folgenden Tabelle spiegeln sich die Ergebnisse von PCR-Untersuchungen wieder. Ziel dieser Untersuchungen war es, aufzuzeigen, dass die erfindungsgemäße Kultivierung in einer erfindungsgemäßen gemäß Beispiel 1 und 2 hergestellten Biomatrix zu einer Redifferenzierung von Chondrocyten führt. Aus der Tabelle wird ersichtlich, dass ausgehend von einer P0-Kultur im Verlaufe der darauffolgenden konventionell durchgeführten Passagen P1 und P2 die Fähigkeit der Chondrocyten verloren geht, Kollagentyp II herzustellen. Die Zellen dedifferenzieren im Verlaufe dieser Passage. Eine P2-Passage in einer erfindungsgemäßen Biomatrix führt dagegen zur Redifferenzierung dedifferenzierter Chondrocyten, was an der wiedergewonnenen Fähigkeit, Kollagentyp II herzustellen, belegt wird.

**Tabelle: Ergebnisse der PCR-Untersuchungen: Aus den Chondrocyten der einzelnen Kulturen wurde die RNA isoliert und in cDNA umgeschrieben.**

| | PO-2D | P1-2D | P2-2D | P2-Kollagen 2x10⁶/ml |
|---|---|---|---|---|
| | 3 Wochen | 2 Wochen | 3 Wochen | 3 Wochen |
| β2Microglobulin | + | + | + | + |
| Kollagen Typ I | + | + | + | + |
| Kollagen Typ II | + | - | - | + |
| Kollagen Typ XI | + | + | + | + |
| Bmp 2 | + | + | + | + |
| Bmp 4 | + | + | + | + |
| Bmp 7 | - | - | - | - |

Die Expression (Transcription) Chondrocyten-spezifischer Gene wurde durch PCR-Amplifikation mit geeigneten Primern und anschließender Gelelektrophorese getestet:
- β2Mikroglobulin ist ein konstitutiv exprimiertes Gen und dient als Positivkontrolle;
- Kollagen Typ II und XI sind Knorpel-spezifische Kollagen-Typen;
- Bone morphogenetic proteins (Bmp) 2,4 und 7 spielen eine Rolle bei der Differenzierung des Knorpelgewebes.

## Patentansprüche

1. Verfahren zur Vermehrung von differenzierten Knorpelzellen, wobei die differenzierten Knorpelzellen unter Erhalt ihrer Differenzierung eingebettet in einer dreidimensionalen, gelartigen Biomatrix, enthaltend 1,5 bis 4 mg/ml Kollagen aus Rattenschwänzen in gepuffertem serum-haltigen Zellkulturmedium, kultiviert werden, wobei die Biomatrix herstellbar ist, indem aus einem Gewebe isolierte Kollagenfasern in 0,1%-iger Essigsäurelösung 3 bis 14 Tage bei 2 bis 10°C gerührt, nicht gelöste Kollagenanteile abzentrifugiert und die erhaltene fertige Kollagenlösung mit einem Kollagengehalt von 3 mg/ml bis 8 mg/ml mit einer Lösung, enthaltend Zellkulturmedium, Serum und Puffer, bei 2 bis 10°C gemischt und anschließend bei höherer Temperatur, Raumtemperatur bis 37°C, geliert wird.

2. Verfahren zur Überprüfung der Funktion, Morphologie und/oder des Differenzierungsstatus von Knorpelzellen, wobei die zu überprüfenden Knorpelzellen in eine dreidimensionale, gelartigen Biomatrix, enthaltend 1,5 bis 4 mg/ml Kollagen aus Rattenschwänzen in gepuffertem serum-haltigen Zellkulturmedium, eingebracht, in dieser kultiviert und gleichzeitig oder danach überprüft werden, wobei die Biomatrix herstellbar ist, indem aus einem Gewebe isolierte Kollagenfasern in 0,1%-iger Essigsäurelösung 3 bis 14 Tage bei 2 bis 10°C gerührt, nicht gelöste Kollagenanteile abzentrifugiert und die erhaltene fertige Kollagenlösung mit einem Kollagengehalt von 3 bis 8 mg/ml mit einer Lösung, enthaltend Zellkulturmedium, Serum und Puffer, bei 2 bis 10°C gemischt und anschließend bei höherer Temperatur, Raumtemperatur bis 37°C, geliert wird.

3. Verfahren nach einem der vorhergehenden Ansprüche, wobei die Knorpelzellen im Anschluss an die Kultivierung in der dreidimensionalen, gelartigen Biomatrix aus dieser herausgelöst und in einer herkömmlichen, vorzugsweise zweidimensionalen, Zellkultur weiter kultiviert werden.

4. Verfahren zur Herstellung eines Knorpelersatzes, wobei Knorpelzellen in eine dreidimensionale, gelartige Biomatrix, enthaltend 1,5 bis 4 mg/ml Kollagen aus Rattenschwänzen in gepuffertem serum-haltigen Zellkulturmedium, eingebracht und in dieser so kultiviert werden, dass ein transplantierbarer Knorpelersatz gewonnen wird, wobei die Biomatrix herstellbar ist, indem aus einem Gewebe isolierte Kollagenfasern in 0,1%-iger Essigsäurelösung 3 bis 14 Tage bei 2 bis 10°C gerührt, nicht gelöste Kollagenanteile abzentrifugiert und die erhaltene fertige Kollagenlösung mit einem Kollagengehalt von 3 bis 8 mg/ml mit einer Lösung, enthaltend Zellkulturmedium, Serum und Puffer, bei 2 bis 10°C gemischt und anschließend bei höherer Temperatur, Raumtemperatur bis 37°C, geliert wird.

5. Verfahren nach Anspruch 4, wobei die Knorpelzellen nach Kultivierung in der dreidimensionalen, gelartigen Biomatrix aus dieser herausgelöst und in höherer Zelldichte weiter kultiviert werden, so dass ein transplantierbarer Knorpelersatz gewonnen wird.

6. Verfahren zur Herstellung einer Biomatrix für die Kultivierung von Knorpelzellen enthaltend die Schritte Isolieren von kollagenhaltigen Rattenschwänzen, Überführen des kollagenhaltigen Gewebes in 0,1%-ige Essigsäurelösung, Inkubieren des in die Essigsäurelösung überführten Kollagengewebes bei 2 bis 10°C, Abzentrifugieren nicht gelöster Kollagenanteile, Mischen der erhaltenen Kollagenlösung mit einem Kollagengehalt von 3 bis 8 mg/ml bei 2 bis 10°C mit einer Lösung, enthaltend Knorpelzellen, Zellkulturmedium, Serum und Puffer, in einem Verhältnis von 1:1 und Gelieren der gemischten Lösung durch Erhöhung der Temperatur.

7. Biomatrix, herstellbar nach dem Verfahren gemäß Anspruch 6.

## Claims

1. Process for the multiplication of differentiated cartilage cells, the differentiated cartilage cells embedded, while retaining their differentiation, in a three-dimensional, gel-type biomatrix containing 1.5 to 4 mg/ml of collagen from rats tails in buffered serum-containing cell culture medium being cultivated, the biomatrix being preparable by collagen fibres isolated from a tissue being stirred in 0.1 % acetic acid solution for 3 to 14 days at 2 to 10°C, non-dissolved collagen parts being removed by centrifuging and the finished collagen solution obtained having a collagen content of 3 mg/ml to 8 mg/ml being mixed with a solution containing cell culture medium, serum and buffer at 2 to 10°C and subsequently gelled at an elevated temperature, room temperature to 37°C.

2. Process for verifying the functioning, morphology and/or differentiation status of cartilage cells, the cartilage cells to be examined being introduced into a three-dimensional, gel-type biomatrix containing 1.5 to 4 mg/ml of collagen from rats tails in buffered serum-containing cell culture medium, cultivated therein and simultaneously or subsequently examined, the biomatrix being preparable by collagen fibres isolated from a tissue being stirred in 0.1 % acetic acid solution for 3 to 14 days at 2 to 10°C, non-dissolved collagen parts being removed by centrifuging and the finished collagen solution obtained having a collagen content of 3 mg/ml to 8 mg/ml being mixed with a solution containing cell culture medium, serum and buffer at 2 to 10°C and subsequently gelled at an elevated temperature, room temperature to 37°C.

3. Process according to one of the preceding claims, the cartilage cells being removed by dissolution, subsequent to cultivation in the three-dimensional gel-type biomatrix from the latter and cultivated further in a conventional, preferably two-dimensional cell culture.

4. Process for the preparation of a cartilage substitute, the cartilage cells being introduced into a three-dimensional, gel-type biomatrix containing 1.5 to 4 mg/ml of collagen from rats tails in buffered serum-containing cell culture medium and cultivated therein in such a way that a transplantable cartilage substitute is obtained, the biomatrix being preparable by collagen fibres isolated from a tissue being stirred in 0.1 % acetic acid solution for 3 to 14 days at 2 to 10°C, non-dissolved collagen parts being removed by centrifuging and the finished collagen solution obtained having a collagen content of 3 mg/ml to 8 mg/ml being mixed with a solution containing cell culture medium, serum and buffer at 2 to 10°C and subsequently gelled at an elevated temperature, room temperature to 37°C.

5. Process according to claim 4, the cartilage cells, after cultivation in the three-dimensional gel-type biomatrix, being removed therefrom by dissolution and cultivated further in a higher cell density such that a transplantable cartilage substitute is obtained.

6. Process for the preparation of a biomatrix for the cultivation of cartilage cells containing the steps of isolating from collagen-containing rats tails, transferring the collagen-containing tissue into 0.1 % acetic acid solution, incubating the collagen tissue transferred into the acetic acid solution at 2 to 10°C, removing non-dissolved collagen parts by centrifuging, mixing the collagen solution obtained with a collagen content of 3 mg/ml to 8 mg/ml at 2 to 10°C with a solution containing cartilage cells, cell culture medium, serum and buffer in a ratio of 1:1 and gelling the mixed solution by increasing the temperature.

7. Biomatrix preparable according to the process of claim 6.

## Revendications

1. Procédé permettant la multiplication des cellules cartilagineuses différenciées, dans lequel les cellules cartilagineuses différenciées sont cultivées, en préservant leur différenciation par incorporation dans une biomatrice de type gel tridimensionnelle contenant de 1,5 à 4 mg/ml de collagène provenant de queues de rats, dans un milieu de culture cellulaire tamponné contenant du sérum, la biomatrice pouvant être produite en agitant des fibres de collagènes, isolées à partir d'un tissu, dans une solution d'acide acétique à 0,1 % durant 3 à 14 jours à une température comprise entre 2 et 10°C, en centrifugeant les parties de collagène non dissoutes et en mélangeant la solution de collagène finie obtenue, présentant une teneur en collagène allant de 3 mg/ml à 8 mg/ml, avec une solution contenant un milieu de culture cellulaire, du sérum et un tampon à une température comprise entre 2 et 10°C puis en la transformant en un gel à une température plus élevée allant de la température ambiante à 37°C.

2. Procédé permettant le contrôle de la fonction, de la morphologie et/ou du stade de différenciation des cellules cartilagineuses, dans lequel les cellules cartilagineuses à contrôler sont insérées dans une biomatrice de type gel tridimensionnelle contenant de 1,5 à 4 mg/ml de collagène provenant de queues de rat dans un milieu de culture cellulaire tamponné contenant du sérum, cultivées et contrôlées simultanément ou ultérieurement dans ladite biomatrice, celle-ci pouvant être obtenue en agitant des fibres de collagènes isolées à partir d'un tissu dans une solution d'acide acétique à 0,1 % durant 3 à 14 jours à une température comprise entre 2 et 10°C, en centrifugeant les parties de collagène non dissoutes et en mélangeant la solution de collagène finie obtenue, présentant une teneur en collagène allant de 3 mg/ml à 8 mg/ml, avec une solution contenant un milieu de culture cellulaire, du sérum et un tampon à une température comprise entre 2 et 10°C puis en la transformant en un gel à une température plus élevée allant de la température ambiante à 37°C.

3. Procédé selon l'une quelconque des revendications précédentes, dans lequel les cellules cartilagineuses après la culture dans la biomatrice de type gel tridimensionnelle sont éliminées de celle-ci et sont cultivées par la suite dans une culture cellulaire traditionnelle, de préférence bidimensionnelle.

4. Procédé permettant la fabrication d'un substitut de cartilage, dans lequel les cellules cartilagineuses sont insérées dans une biomatrice de type gel tridimensionnelle contenant de 1,5 à 4 mg/ml de collagène provenant de queues de rat dans un milieu de culture cellulaire tamponné contenant du sérum et cultivées dans ladite biomatrice, de sorte à obtenir un substitut de cartilage transplantable, la biomatrice pouvant être produite en agitant des fibres de collagènes isolées à partir d'un tissu dans une solution d'acide acétique à 0,1 % durant 3 à 14 jours à une température comprise entre 2 et 10°C, en centrifugeant les parties de collagène non dissoutes et en mélangeant la solution de collagène finie obtenue, présentant une teneur en collagènes allant de 3 mg/ml à 8 mg/ml, avec une solution contenant un milieu de culture cellulaire, du sérum et un tampon à une température comprise entre 2 et 10°C puis en la transformant en un gel à une température plus élevée allant de la température ambiante à 37°C.

5. Procédé selon la revendication 4, dans lequel les cellules cartilagineuses, après culture dans la biomatrice de type gel tridimensionnelle, sont éliminées de celle-ci et sont ensuite cultivées en une densité cellulaire plus élevée, de sorte à obtenir un substitut de cartilage transplantable.

6. Procédé permettant la fabrication d'une biomatrice pour la culture de cellules cartilagineuses présentant les étapes consistant à isoler des queues de rat contenant du collagène, à transformer le tissu contenant du collagène dans une solution de type acide acétique à 0,1 %, à incuber le tissu de collagène transformé dans la solution d'acide acétique à une température comprise entre 2 et 10°C, à centrifuger les parties de collagène non dissoutes, à mélanger la solution de collagène obtenue, présentant une teneur en collagène de 3 mg/ml à 8 mg/ml, à une température comprise entre 2 et 10°C avec une solution contenant des cellules cartilagineuses, un milieu de culture cellulaire, du sérum et un tampon, selon un rapport de 1 : 1 et à transformer en un gel la solution mélangée par élévation de la température.

7. Biomatrice pouvant être produite d'après le procédé selon la revendication 6.
